# DEMANDE DE BREVET EUROPEEN

(11) **EP 2 260 845 A2**
(43) Date de publication de la demande: **15.12.2010**
(21) Numéro de dépôt: 10184434.8
(22) Date de dépôt: 23.06.2003
(51) Int. Cl.: A61K 31/352, A61K 31/353, A61K 31/015, A61K 45/06, A61K 31/201, A61K 31/202, A61K 9/20, A61K 9/48, A61K 8/86, A61Q 7/00, A61K 8/46, A61K 8/49

(54) **Utilisation de polyphénol pour le traitement de l'alopécie**

(30) Priorité: 21.06.2002 FR 0207763; 21.06.2002 FR 0207764; 21.06.2002 FR 0207765
(62) Demande divisionnaire de: 03760772.8
(71) Demandeur: L'Oréal, 75008 Paris (FR); Nestec S.A., 1800 Vevey (CH)
(72) Inventeur: Duranton, Albert, 78600, MAISONS-LAFFITTE (FR); Breton, Lionel, 78000, Versailles (FR)
(74) Mandataire: Le Coupanec, Pascale A.M.P.

(57) **Abrégé**

La présente invention concerne l'utilisation de polyphénol(s) choisi(s) parmi les flavonols, anthocyanines, flavanols, proanthocyanidines et flavanones, et stilbènes pour la préparation d'une composition orale utile dans le traitement ou la prévention des désordres de l'unité pilo-sébacée.

## Description

La présente invention concerne l'utilisation d'acide(s) gras, de polyphénol et/ou d'extraits en comportant, éventuellement en association avec de la taurine dans des compléments alimentaires pour le traitement et la prévention de ces mêmes désordres.

Certaines altérations physiologiques apparaissent avec l'âge, les variations saisonnières, le stress et les agressions atmosphériques. Il s'agit notamment, de la réduction de la densité des cheveux au cours du vieillissement, le nombre et le diamètre des tiges pilaires diminuant. En particulier certains sujets développent le phénomène d'alopécie.

Pour prévenir les altérations de la chevelure se manifestant principalement avec l'âge, on a jusqu'à présent utilisé des acides aminés essentiels, reconnus indispensables comme nutriment pour la synthèse de la kératine dans le bulbe pileux. Ainsi la méthionine, la cystine et la cystéine sont connues pour avoir un impact direct sur le métabolisme du follicule pileux. Toutefois ces acides aminés essentiels agissent sur la synthèse des protéines qui n'est pas le seul mécanisme intervenant dans le phénomène de l'alopécie.

Parmi les causes de l'alopécie, on a en effet déterminé qu'une altération du tissu conjonctif périfolléculaire se traduisait par une rigidification de la gaine conjonctive qui expliquerait la miniaturisation du follicule pileux, signe de vieillissement de l'unité pilosébacée.

De plus, ces altérations du cheveu sont souvent accompagnées d'altération de l'état du scalp, telles la production abondante de sébum. L'hypersécrétion sébacée ou séborrhée et ses conséquences, l'acné par exemple, se manifestent fréquemment au moment de la puberté, mais peuvent se poursuivre à l'âge adulte, notamment chez les femmes, pour des causes hormonales.

Ces désordres peuvent se produire conjointement, à différents degrés, chez un même individu.

Pour lutter contre le phénomène d'alopécie, qui caractérise le follicule pileux, on a préconisé l'utilisation de médicaments inhibiteurs du métabolisme du collagène. Il est connu d'utiliser notamment le minoxidil, et à ce jour, le mécanisme d'action du minoxidil, connu pour pouvoir lutter contre le processus de miniaturisation du follicule pileux, sans être un anti-androgène, est encore inconnu.

Pour lutter contre l'hypersécrétion sébacée, des traitements par voie locale ont été proposés dont l'isotrétinoine, mais ce traitement n'est pas dénué d'effets secondaires sérieux.

On a également préconisé l'utilisation d'anti-androgènes contre l'alopécie et l'hypersécrétion sébacée, par voie systémique. Ce type de traitement n'est cependant pas dénué d'effets secondaires sérieux en particulier sur les organes sexuels.

Pour sa part, le document WO 99/22728 décrit de nombreux composés dont les acides gras, notamment, pour des utilisations thérapeutiques. Toutefois, les médicaments présentent des inconvénients liés aux risques inhérents à leur utilisation, dans la mesure où les médicaments sont des xénobiotiques. En outre, les médicaments ont en général un spectre d'action très ciblé, alors même que les causes de l'altération de l'unité pilo-sébacée sont multiples.

Par ailleurs, la taurine est décrite comme un activateur cellulaire pour la régulation des cellules capillaires et proposée dans des compositions de stimulants capillaires d'application topique, dans le document WO 02 24189. Néanmoins, la taurine utilisée comme activateur cellulaire par voie topique a des effets limités du fait que la perte d'activité cellulaire peut être due à de multiples causes de l'alopécie. Si ces causes persistent, les effets temporaires d'une application topique d'activateur cellulaire sont limités. De plus, les compositions à usage topique présentent de leur côté les inconvénients liés à l'application locale. La fréquence des applications est généralement plus importante et l'application de ces compositions sur une surface à traiter de taille importante, peut requérir un certain temps.

On constate donc qu'il subsiste un besoin d'actifs que l'on peut administrer par voie orale, efficaces dans le traitement et/ou la prévention des signes du vieillissement du cheveu et/ou des désordres fonctionnels de l'unité pilo-sébacée, et notamment de l'alopécie, et dépourvus d'effets secondaires. L'unité pilo-sébacée comporte un follicule pileux et sa glande sébacée.

De façon surprenante, la Demanderesse a mis en évidence d'une part que la taurine a un intérêt pour réguler l'altération du tissu conjonctif du follicule pileux, et peut ainsi être avantageusement utilisée dans le traitement et la prévention du vieillissement de l'unité pilo-sébacée et/ou de l'alopécie. Elle a en effet pu observer que la taurine réduit l'incorporation de proline sans altérer celle de la leucine ; ceci montre l'intérêt de la taurine pour réduire spécifiquement l'accumulation de collagène, sans altérer la synthèse globale des protéines.

D'autre part, la Demanderesse a également constaté que des extraits riches en polyphénols et/ou en acides gras, utilisés par voie orale, notamment à titre de complément alimentaire, ont une activité bénéfique sur les désordres se manifestant au niveau de l'unité pilo-sébacée. Des compositions orales comportant des polyphénols et/ou des acides gras peuvent notamment prévenir l'activation cutanée de la testostérone (intacrine), et l'augmentation de la fonction sébacée qui en résulte, et ceci sans effet général sur l'appareil génital ou les fonctions sexuelles.

Par ailleurs, la Demanderesse a noté que le fait de privilégier une administration par voie orale, permettait d'obtenir un effet anti-chute des cheveux sans induire un effet stimulateur au niveau de la pousse du système pileux autre que le système capillaire. Elle a ainsi constaté qu'une administration par voie orale, des matières actives considérées selon l'invention s'avérait particulièrement efficace pour maintenir une belle chevelure en agissant sur la densité capillaire c'est-à-dire le nombre de cheveux par cm² de cuir chevelu et en réduisant l'hétérogénéité des diamètres capillaires.

La présente invention vise selon un de ses aspects l'utilisation de polyphénol(s) choisi(s) parmi les flavonols, anthocyanines, flavanols, proanthocyanidines et flavanones, et stilbènes et/ou d'acide(s) gras choisi(s) parmi les acides gras polyinsaturés essentiels n-6 et n-3, comportant entre 18 et 22 atomes de carbone, ainsi que leurs esters, et leurs mélanges et/ou d'un extrait en comportant, pour la préparation d'une composition orale, notamment d'un complément alimentaire utile dans le traitement ou la prévention des désordres de l'unité pilo-sébacée, en particulier utile pour réduire ou prévenir la séborrhée voire pour réduire ou prévenir la chute des cheveux.

Ces compositions orales sont notamment avantageuses pour réduire ou prévenir le métabolisme excessif des androgènes au niveau de la peau et/ou réduire ou prévenir l'impact de la testostérone sur l'unité pilo-sébacée.

Plus particulièrement, les polyphénols sont choisis parmi les flavones, flavonols, isoflavones, anthocyanines, flavanols, proanthocyanidines et flavanones, et stilbènes et les acides gras sont choisis parmi les acides gras polyinsaturés essentiels n-6 et n-3, comportant entre 18 et 22 atomes de carbone, ainsi que leurs esters, et leurs mélanges.

Les compositions pour l'absorption orale selon l'invention peuvent comprendre 0,01 à 30 % en poids de taurine, et/ou d'hypotaurine et/ou de leurs sels acceptables, en association avec 0,1 à 50 % en poids d'extraits comportant au moins un polyphénol, et notamment avec 0,1 à 25 % en poids, notamment 0,1 à 20 % en poids, voire 0,1 à 15 % en poids de catéchines.

Les compositions pour l'absorption orale selon l'invention peuvent notamment posséder de la taurine, et/ou hypotaurine et/ou leurs sels acceptables, en association avec des polyphénols dans un rapport pondéral polyphénol/taurine au moins égal à 0,5, en particulier supérieur ou égal à 0,75, notamment supérieur ou égal à 1.

Un autre aspect de l'invention concerne une composition pour l'absorption orale comportant au moins 0,01 à 30 % en poids de taurine, et/ou d'hypotaurine et/ou de leurs sels acceptables, en association avec 0,01 à 10 % en poids d'acides gras.

Selon un mode de réalisation particulier, les compositions orales de l'invention sont des compléments alimentaires.

Selon un autre de ses aspects, l'invention se rapporte à un procédé cosmétique de traitement et de prévention des désordres de l'unité pilo-sébacée par l'administration orale d'au moins un acide gras, un polyphénol ou d'un extrait en comportant éventuellement en association avec de la taurine et/ou de l'hypotaurine et/ou leurs sels acceptables.

Dans un mode de réalisation particulier, on administre la taurine, l'hypotaurine ou leurs sels acceptables à une dose de 0,5 à 4000 mg par jour en équivalent taurine, le ou les acide(s) gras à une dose de 0,5 à 5400 mg/j et/ou le ou les polyphénol(s) à une dose de 0,5 à 2000 mg/j.

Selon l'invention, les effets attendus sont atteints sans les effets indésirables d'un médicament, de façon peu coûteuse par rapport au prix d'un traitement par un médicament. La prise orale du ou des actifs permet un effet plus constant, sans que l'on soit obligé de réitérer les applications.

Plus particulièrement, on lève les limitations d'efficacité quand cette prise orale est réalisée chez des sujets chez qui on a détecté un signe précurseur de désordre fonctionnel de l'unité pilo-sébacée notamment de l'alopécie, comme un état de réticulation excessive du conjonctif périfolliculaire, par exemple par histologie ou par macro-photographie du scalp.

L'invention sera mieux comprise à la lecture de la description détaillée et des exemples suivants.

### LA TAURINE, L'HYPOTAURINE

A titre d'actif selon l'invention, on peut utiliser la taurine et/ou l'hypotaurine, qui en est un métabolite. On peut utiliser également leurs sels acceptables dans de telles compositions orales. Dans la mesure où les compositions selon l'invention sont destinées à être administrées à un sujet, ces sels sont bien entendu choisis pour leur totale innocuité. A ce titre, conviennent tout particulièrement à l'invention les sels alcalins ou alcalinoterreux, en particulier les sels de magnésium, manganèse, fer II ou zinc.

Selon l'invention, l'hypotaurine, ou la taurine sont utilisées à des doses journalières allant de 0,5 à 4000 mg par jour, de préférence 10 à 500 mg par jour. De façon encore préférée, la dose journalière est d'environ 50 à 150 mg par jour. Les doses indiquées dans la présente description sont les doses en équivalent taurine.

### LES POLYPHENOLS

A titre de polyphénol selon l'invention, on peut utiliser tout polyphénol alimentaire. Ces composés sont en général issus de plantes, et leurs structures sont classées selon la nature du squelette hydrocarboné (Laura Bravo Nutrition Review 1998 56 pp 317-333, Scalbert A. Williamson GJ Nutr2000 130 2073s 2085S).

Selon l'invention, on entend plus particulièrement par polyphénols, les composés de type flavonoïdes, c'est à dire les flavones, flavonols, isoflavones, anthocyanines, flavanols, proanthocyanidines et flavanones, et stilbènes.

Conviennent plus particulièrement les flavonols, anthocyanines, flavanols, proanthocyanidines et flavanones et stilbènes.

Les principaux flavanols seront choisis parmi les catéchines, gallocatéchines. Les procyanidines sont des polymères de flavonols présents sous formes de mélanges de polymères de faible degré. Ils peuvent être associés à des catéchines dans les extraits végétaux.

De préférence, on utilise des polyphénols ou mélanges de polyphénols choisis parmi la catéchine, l'épicatéchine, l'épigallocatéchine-3-O-gallate, l'épigallocatéchine, l'épicatéchine-3-gallate, les procyanidines, proanthocyanidines et leurs mélanges.

Il est particulièrement avantageux d'utiliser des monomères catéchines en mélange le cas échéant avec des oligomères procyanidines (OPC). Ainsi, les polyphénols mis en oeuvre selon l'invention ne peuvent être constitués que de monomères catéchines.

Ces apports en polyphénols peuvent être faits à partir de composés isolés et/ou à partir d'extraits végétaux et leurs mélanges.

On pourra selon l'invention utiliser des extraits végétaux pouvant apporter l'ensemble de ces polyphénols.

Plus particulièrement, les catéchines sont très abondantes dans le thé (Camellia Simensis), et le raisin (Vitis Vinifera) et autres fruits (pomme, poire, pomme de pin (Pinus Maritima). Les breuvages (vin, bière, thé) et le chocolat (Théobroma Cacao) sont des sources pouvant constituer des apports en catéchines selon l'invention.

Ces polyphénols pourront être utilisés seuls ou utilisés sous forme de mélanges, et peuvent être ingérés sous différentes formes de compléments nutritionnels (dragée, gels, poudres solubles, gélules, capsules, aliments enrichis...).

Ces polyphénols alimentaires peuvent être utilisés à des doses de 0,5 à 2000 mg/jour, notamment de 0,5 à 1000 mg/jour, de préférence de 20 à 300 mg/j.

En particulier, ces polyphénols peuvent être administrés par voie orale à des doses dites nutritionnelles c'est-à-dire équivalentes aux doses absorbées par un sujet humain soumis à un régime alimentaire équilibré.

A titre d'exemple, on peut citer un extrait de pépins de raisin à 40 % OPC, un extrait de vin rouge à 30 % de polyphénols totaux et/ou un extrait de thé vert à 30 % de catéchines.

Les oligomères procyanidines (OPC) peuvent être utilisés à des doses de 0,5 à 1000 mg/j, de préférence 20 à 250 mg/j. Ils peuvent être apportés par un extrait de pépins de raisin, que l'on dose selon son titre OPC. A titre d'exemple, pour un extrait de pépin de raisin à 40 % OPC, ci-dessus, on l'utilise à une dose de 150 mg/j, soit 60 mg/j OPC.

Par ailleurs, les catéchines peuvent être utilisées à des doses de 0,5 à 1000 mg/j, de préférence 20 à 300 mg/j. Elles peuvent être apportées par exemple par un extrait de thé vert à 30 % de catéchines, la dose d'extrait étant alors de l'ordre de 375 mg/j, soit 112,5 mg/j de catéchines.

Comme exemple de doses journalières en polyphénols, on peut citer des doses journalières d'un extrait de vin rouge riche en composants polyphénoliques (600 mg de poudre d'extrait de vin rouge : 12 mg OPC/personne/jour soit 18 mg polyphénols totaux), des doses journalières d'un extrait de pépin de raisin riche en composants polyphénoliques (300 mg de poudre d'extrait de vin rouge : 18 mg OPC/personne/jour soit 27 mg polyphénols totaux), des doses journalières extrait de thé vert riche en composants polyphénoliques (225 mg de poudre d'extrait de Thé vert : 67,5 mg de catéchines/ jour).

Les polyphénols peuvent être choisis dans une des catégories ci-dessus, et on peut aussi opérer des mélanges. Les compositions de compléments alimentaires peuvent comporter 0,01 à 10 % en poids d'au moins un polyphénol.

Dans le cas où ces polyphénols sont administrés en association avec la taurine, et/ ou l'hypotaurine, ils peuvent être administrés à raison de 0,1 à 50 % en poids pour 0,01 à 30 % en poids de taurine et/ou l'hypotaurine et/ou leurs sels acceptables.

La Demanderesse a notamment mis en évidence que l'administration par voie orale d'une dose de 37 mg/J/kg d'un concentré de vin rouge, ce qui équivaut à une dose de 220 mg/J/kg pour un sujet humain de 60 kg, avait un effet efficace sur la chute des cheveux sans manifestation d'effet secondaire indésirable au niveau de la prostate. Ceci est notamment illustré dans les exemples ci-après.

Comme précisé précédemment, il est avantageux d'utiliser les polyphénols en association avec la taurine dans un rapport pondéral polyphénol/taurine au moins égal à 0,5, en particulier supérieur ou égal à 0,75, notamment supérieur ou égal à 1. Plus particulièrement ce rapport pondéral peut varier de 0,5 à 2, notamment de 0,75 à 1,5 voire de 0,9 à 1,3 en particulier être de l'ordre de 1,2.

S'avèrent également tout particulièrement avantageuses des compositions conformes à l'invention comprenant de 0,01 à 30 % en poids de taurine et/ou d'hypotaurine et/ou de leurs sels acceptables et notamment de taurine, en associant avec 0,1 à 25 % en poids de catéchines, notamment 0,1 à 20% en poids de catéchines présentes sous la forme d'un extrait végétal ou d'un mélange d'extraits végétaux.

Dans le cas particulier des catéchines, ces composés peuvent être associés à la taurine, l'hypotaurine et/ou leurs sels dans un rapport pondéral catéchine /taurine au moins égal à 0,4, notamment supérieure à 0,7, voire compris entre 0,7 et 1,5.

### LES ACIDES GRAS

A titre d'acides gras selon l'invention, on entend les acides gras polyinsaturés, c'est-à-dire tous les acides gras avec doubles liaisons interrompues cis, cis-méthylène.

Les acides gras polyinsaturés alimentaires sont définis selon la longueur de la chaîne carbonée et la position de la double liaison. On range actuellement les acides gras essentiels en deux groupes (ω3 et ω6) caractérisés par la position de l'insaturation la plus proche du groupe méthyle terminal.

Conviennent tout particulièrement à l'invention, les acides gras de deux familles d'acides gras polyinsaturés essentiels des familles d'acides gras n-6 et n-3, comportant entre 18 et 22 atomes de carbone ainsi que leurs esters et leurs mélanges. Ces acides gras présentent en effet l'intérêt d'être autorisés selon les normes alimentaires.

De préférence, ces acides gras ne sont pas associés à des terpènes ou dérivés terpéniques.

Pour les acides gras polyinsaturés des séries n-6, appelés « oméga-6 », on peut citer le premier, l'acide linoléique, à 18 atomes de carbone et deux insaturations : (18 :2ω6), et l'acide γ-linolénique (18 :3ω6) est aussi un acide gras particulièrement intéressant selon l'invention.

Les sources d'acide γ-linolénique seront choisies parmi des huiles végétales (huiles d'onagre, de bourrache, de pépin de cassis et de chanvre), les extraits de la spiruline, Spirula. maxima et S. platensis.

Pour les acides gras polyinsaturés des séries n-3, appelés « oméga-3 », le premier est l'acide alpha-linolénique (18 :3ω3) ; l'acide stéaridonique (C18 :4 n-3) est aussi un acide gras particulièrement intéressant dans l'invention.

Les huiles végétales de noix (Juglans regia) et de soja (Glycina max) par exemple, sont riches en acides gras polyinsaturés de la série oméga 3 au même titre que les huiles de poisson.

Les acides gras polyinsaturés ω3 se retrouvent, via la chaîne alimentaire, dans le zooplanton, les crustacés / mollusques et les poissons.

Les huiles de poissons constituent la principale source industrielle d'EPA (acide eicosapentaénoïque = 20:5 ω3) et le DHA (acide docosahéxaénoïque = 22 :6 ω3). Cependant, les biomasses de microalgues peuvent aussi constituer une matière première d'extraction des ω3.

La qualité nutritionnelle des microalgues peut être améliorée par un choix judicieux de souches et par une orientation métabolique liée aux conditions de culture.

L'intérêt pour les microalgues est d'autant plus grand qu'elles synthétisent des acides gras, tel l'EPA et le DHA.

De préférence, on utilise l'acide linoléique, l'acide γ-linolénique, l'acide linolénique, l'acide stéaridonique, la crocétine et l'acide 5, 8, 11, 14 éicosatétrainoïque et leurs mélanges ou des extraits les comportant. Ainsi, le ou les acides gras et/ou le ou les extraits peuvent être utilisés seuls ou en mélanges.

Les doses journalières préconisées selon l'invention sont, pour les acides gras comprises entre 0,5 et 3500 mg/j, notamment entre 5 et 1500 mg/j.

Les doses journalières préconisées selon l'invention sont, pour les acides gras n-3 comprises entre 0,5 et 2500 mg/j, de préférence 5 à 360 mg/j, et pour les acides gras n-6 comprises entre 0,5 et 2600 mg/j, de préférence 5 à 1200 mg/j.

Les acides gras peuvent être choisis dans une des catégories ci-dessus, et on peut aussi opérer des mélanges. Les compositions orales peuvent comporter 0,01 à 10 % en poids d'au moins un acide gras.

Dans le cas d'une association avec la taurine et/ou l'hypotaurine et/ou de leurs sels acceptables, les compositions selon l'invention peuvent comprendre de 0,01 à 30 % en poids de taurine et/ou d'hypotaurine et/ou de leurs sels acceptables avec 0,01 à 10 % en poids d'acides gras.

### AUTRE(S) ACTIF(S)

Les actifs considérés selon l'invention, à savoir la taurine, l'hypotaurine ou leurs sels, les acides gras, les polyphénols et leurs mélanges peuvent être associés à un ou plusieurs autres actifs tels que notamment des vitamines et antioxydants, éventuellement sous forme de complexes.

Au sens de la présente invention, le terme actif signifie que le composé considéré, par exemple la taurine, est mis en oeuvre pour manifester l'activité biologique et chimique qui lui est propre et non pas pour une fonction de type véhicule ou excipient.

Bien entendu, les compositions selon l'invention peuvent contenir plusieurs actifs.

A titre d'actifs utilisables, on peut citer le zinc et ses sels notamment le sulfate et le glucanate, les vitamines B5, B6, B8, C, E, ou PP, le β-carotène et les caroténoïdes, les extraits d'ail sous forme de sulfure d'allyle ou d'ajoen par exemple, le sélénium, la curcumine, les curcuminoïdes, la niacine, l'acide lithospermique, et l'adénosine.

En particulier, on peut utiliser un complexe anti-oxydant comprenant les vitamines C et E, du zinc ou ses sels, le sélénium et au moins un caroténoïde, notamment le caroténoïde choisi parmi le β-carotène, le lycopène, la zéaxanthine et la lutéine.

On préfère un complexe anti-oxydant comportant par exemple de 100 à 150 mg de vitamine C pour 80 à 120 µg de Sélénium, 20 à 50 mg de vitamine E, 10 à 40 mg de zinc et 3 à 10 mg de β-carotène.

Toutefois, les compositions selon l'invention peuvent avantageusement contenir moins de 1 % en poids de vitamine C, voire en être exempte.

Les actifs selon l'invention peuvent en outre être associés à des actifs anti-chute connus, et notamment des composés améliorant encore leur activité sur la repousse et/ou sur le freinage de la chute des cheveux, tels que plus particulièrement les composés suivants :
- les esters d'acide nicotinique, dont plus particulièrement les nicotinates d'alkyle en C3-C6 et notamment le nicotinate de méthyle ou d'hexyle, le nicotinate de benzyle ou de tocophérol ;
- les agents anti-inflammatoires stéroïdiens et non stéroïdiens bien connus dans l'état de la technique et en particulier l'hydrocortisone, ses sels et ses dérivés, l'acide niflumique ;
- les rétinoïdes et plus particulièrement l'acide t-trans rétinoïque appelé encore trétinoïne, l'isotrétinoïne, le rétinol ou la vitamine A et ses dérivés, tels que l'acétate, le palmitate ou le propionate, le motrétinide, l'étrétinate, le t-rans rétinoate de zinc ;
- les agents antibactériens choisis plus particulièrement parmi les macrolides, les pyranosides et les tétracyclines et notamment l'érythromycine ;
- les agents antagonistes de calcium tels que la Cinnarizine et le Diltiazem ;
- des hormones, telles que l'estriol ou des analogues ou la thyroxine et ses sels ;
- des agents antiandrogènes, tels que l'oxendolone, spironolactone, le diéthylstilbestrol ;
- des capteurs de radicaux OH, tels que le diméthylsulfoxyde ;
- des oligosaccharides estérifiés, tels que ceux décrits dans EP-A-0 211 610 et EP-A-0 064 012 ;
- des dérivés d'acide hexosacchariques, tels que ceux décrits dans EP-A-0 375 388, en particulier l'acide glucosaccharique ;
- des inhibiteurs de glycosidase, tels que ceux décrits dans EP-A-0 334 586, en particulier le D-glucaro 1,5-lactam ,
- des inhibiteurs de glycosaminoglycanases et protéoglycanases, tels que ceux cités dans EP-A-0 277 428, en particulier la L-galactono 1 ,4-lactone ;
- des inhibiteurs de tyrosine kinase, tels que ceux décrits dans EP-A-0 403 238, en particulier le 1-amido 1-cyano-(3,4-dihydroxyphényl)éthylène.

On peut également associer avec les actifs de l'invention, éventuellement en mélange avec les autres, des composés tels que le Diazoxyde correspondant au méthyl-3 chloro-7[2H]benzothiadiazine-1,2,4 dioxyde-1-1 ; la Spiroxazone ou 7-(acétylthio)-4',5'-dihydrospiro-[androst 4-ène- 17,2'-(3'H)furan]3 one ; des phospholipides tels que la lécithine ; l'acide salicylique et ses dérivés décrits plus particulièrement dans le brevet français n° 2 581 542, et plus particulièrement les dérivés d'acide salicylique porteurs d'un groupement alcanoyle ayant 2 à 12 atomes de carbone en position 5 du cycle benzénique, des acides hydroxycarboxyliques ou cétocarboxyliques et leurs esters, des lactones et leurs sels correspondants ; l'anthraline, les acides eicosatriynoïque-5,8,11, leurs esters et amides, le minoxidil et ses dérivés, composés décrits dans EP 353 123, EP 356 271, EP 408 442, EP 522 964, EP 420 707, EP 459 890, EP 519 819, US 4 139 619 et US 459 812.

Les composés ci-dessus sont incorporés dans les compositions orales et notamment les compléments alimentaires pour autant que leur utilisation en tant que complément alimentaire soit possible, et leur formulation compatible avec celle des actifs de l'invention. Ces actifs additionnels sont utilisés selon l'invention à des doses compatibles avec leur usage en tant que compléments alimentaires. Ainsi, pour certains composés, on préférera les utiliser par voie topique, en complément des compléments alimentaires de l'invention.

Pour l'ingestion de l'actif ou des actifs, de nombreuses formes de réalisation de compositions orales et notamment de compléments alimentaires sont possibles. Leur formulation est réalisée par les procédés usuels pour produire des dragées, gélules, gels, émulsions, comprimés, capsules ou solutions liquides notamment ampoules buvables, par exemple. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluables à l'eau, dans du soda, des produits laitiers ou dérivés du soja, ou être incorporées dans des barres alimentaires.

Les actifs peuvent être formulés avec les excipients et composants usuels pour de tels compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires sont connus dans ce domaine et ne font pas ici l'objet d'une description détaillée.

Le procédé cosmétique selon l'invention est mis en oeuvre par une prise orale, journalière par exemple, de composition orale ou complément alimentaire qui peuvent être par exemple sous forme de gélules, gels, dragées, émulsions, comprimés, capsules ou ampoules buvables, en quantité et nombre adéquats, selon leur forme, pour que la taurine et/ou l'hypotaurine ou leurs sels acceptables soient ingérés à raison de 0,5 à 4000 mg par jour, de façon préférée 10 à 500 mg par jour, et de façon plus préférée environ 150 mg par jour, en équivalent taurine et/ou que le(s) polyphénol(s) soient ingérés à des doses de l'ordre de 0,5 à 2000 mg/j, et/ou que les acides gras soient ingérés à des doses de 0,5 à 5400mg par jour, préférentiellement de 5 à 1600mg par jour.

Le procédé selon l'invention peut consister en une prise unique mais généralement est appliqué sur une durée prolongée d'au moins 4 semaines, voire 4 à 8 semaines, avec le cas échéant une ou plusieurs périodes d'interruption.

A titre d'exemple, pour l'acide gamma-linolénique, qui peut être apporté par de l'huile de pépin de cassis, on peut prévoir des doses de l'ordre de 10 à 3000 mg/j, de préférence de 50 à 1000mg/j.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans les conditions facilement mises en oeuvre par l'homme de l'art.

Les exemples et figures soumis ci-après sont présentés à titre illustratif et non limitatif du domaine de l'invention.
Figure 1 : Elle représente les résultats de l'ingestion d'acides gras par des hamsters, dans un test CVO.
Figure 2 : Elle représente les résultats de l'ingestion de polyphénols par des hamsters dans un test CVO.
Figure 3 : Elle présente les résultats d'antichute de cheveux constatés chez des sujets traités selon l'invention et des sujets témoins.

### EXEMPLE DE MISE EN EVIDENCE DE L'ACTIVITE DES ACIDES GRAS, ET DES POLYPHENOLS.

Afin de mettre en évidence l'activité de ces composés un test de détection d'activité au niveau d'une formation pilosébacée spécifique a été utilisé : le test du CVO.

Le CVO (organe costo-vertébral) du Hamster est une région cutanée riche en unités pilosébacées (follicules pileux et leurs glandes sébacées). La taille de cette formation est augmentée sous l'action de la testostérone. Le test (Liao S & al. Arch Dermatot Res 2001 Apr :293(4) :200-205) consiste à déterminer l'action anti-androgène de composés sur le CVO, c'est-à-dire à déterminer si les composés empêchent l'action de la testostérone.

### 1/ Test CVO avec DES ACIDES GRAS :

Dans ce test, l'huile de pépin de cassis à 10 % dans l'aliment durant 85 jours est donnée à des Hamsters mâles. On constate que ce complément nutritionnel prévient l'augmentation de la taille du CVO induite par la testostérone.

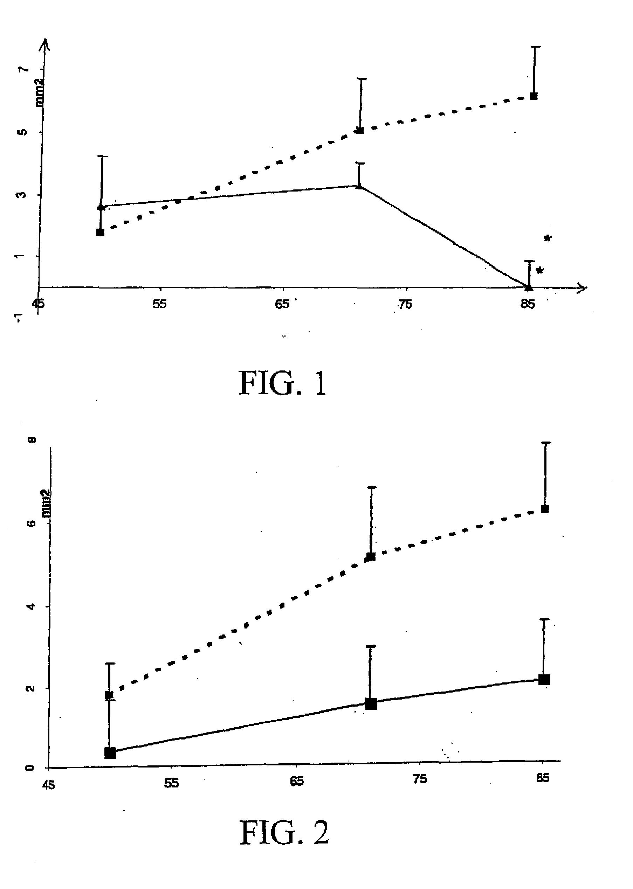

Ainsi, à la figure 1, la courbe en pointillé représente l'évolution de la taille du CVO (mm2) chez des animaux témoins, sans supplémentation, au cours de l'expérience (jours en abscisse): il y a augmentation de la taille du CVO induite par la testostérone. La courbe en trait continu représente l'évolution de la taille du CVO chez des animaux avec supplémentation : cette augmentation est réduite à pratiquement zéro.

### 2/ TEST CVO avec DES POLYPHENOLS

On a fait ingérer à des hamsters un nutriment sous forme de concentré de vin rouge Robertet comportant 18 % de composants flavanoïdes (0,11 g dans 43 g d'aliment) quotidiennement, et on a constaté que la différence CVO droit CVO gauche est pratiquement nulle, à 85 j d'ingestion (figure 2).

Ainsi, à la figure 2, la courbe en pointillé représente l'évolution de la taille du CVO (mm2) chez des animaux témoins, sans supplémentation, au cours de l'expérience (jours en abscisse) : il y a augmentation de la taille du CVO induite par la testostérone. La courbe en trait continu représente l'évolution de la taille du CVO chez des animaux avec supplémentation : cette augmentation est réduite à pratiquement zéro.

### 3/ Absence d'effet secondaire sur la sphère sexuelle :

Chez les hamsters ayant reçu ces compléments nutritionnels par voie orale, on a constaté la répercussion de l'action anti-androgène non seulement sur le CVO, mais également sur les organes sexuels chez des animaux mâles. Dans les deux cas, on a constaté que ces compléments nutritionnels n'altèrent pas le poids des vésicules séminales et de la prostate.

### EXEMPLE DE MISE EN EVIDENCE DE L'ACTIVITE DE LA TAURINE

On a réalisé une étude dans le but d'évaluer, par une méthode de screening, les effets des composés sur la croissance des fibroblastes et la synthèse des constituants majeurs de la matrice extracellulaire. La technique a permis d'étudier et d'évaluer l'intérêt de la taurine sur ce métabolisme cellulaire. La technique a permis d'étudier et d'évaluer l'intérêt de la taurine sur ce métabolisme cellulaire (T. Shigematsu et al. Biochimica et Biophysica Acta 1200 (1994) 79-83).

Un pool de fibroblastes dermiques humains normaux (NHDF pool PF2, utilisé au huitième passage) a été cultivé en conditions standards dans un milieu : DMEM, L-glutamine 2mM, pénicilline/streptomicyne 50 UI/ml/50g/ml, sérum de veau foetal à 0,5 %.

La taurine a été testée à des concentrations de 10 mM et 1 mM, dans un milieu de culture stérile, contre un témoin contrôle non traité.

Les résultats de l'incorporation aux fibroblastes de la thymidine, la proline et la leucine apparaissent dans le tableau I suivant qui présente l'effet de la taurine sur l'incorporation de thymidine, de proline et de leucine dans les macromolécules néosynthétisées par les NHDF en culture in vitro. Les chiffres en gras sont ceux pour lesquels il y a variation significative (sign. stat. pour significativité statistique : p < 0,005). Les résultats sont exprimés en pourcentage du contrôle.

**Tableau 1**

| | Thymidine | | Proline | | Leucine | |
|---|---|---|---|---|---|---|
| | % contrôle | sign.stat | % contrôle | sign.stat | % contrôle | sign.stat |
| Taurine 1 mM | 89 | p>0.05 | 88 | p<0.01 | 100 | p>0.05 |
| Taurine 10 mM | 90 | p>0.05 | 87 | p<0.01 | 105 | p>0.05 |

Il apparaît que la taurine, aux deux concentrations traitées, n'a pas modifié de façon significative l'incorporation par les fibroblastes de la thymidine, représentative de la prolifération cellulaire ni de la leucine, représentative de la synthèse de protéine non collagénique ; en revanche, la taurine a inhibé l'incorporation par les fibroblastes de la proline, de manière significative.

### EXEMPLES DE FORMULATION.

### Exemple 1

### GEL UNIDOSE

| **Principe actif** | **% pds** |
|---|---|
| Taurine | 4 |
| Huile de pépin de cassis | 10 |
| | |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On peut utiliser 200 à 400 ml de ce produit par jour.

### Exemple 2

### GEL UNIDOSE

| **Principe actif** | **% pds** |
|---|---|
| Taurine | 4 |
| Huile de pépin de cassis | 10 |
| Complexe antioxydant | * |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

| | |
|---|---|
| * Le complexe antioxydant comporte 120 mg de vitamine C, 100 µg de sélénium, 30 mg de vitamine E, 20 mg de zinc et 6 mg de β-carotène pour 200 ml de gel. | |

On peut utiliser 200 à 400 ml de ce produit par jour.

### Exemple 3

### CAPSULE

| | **mg/capsule** |
|---|---|
| Taurine | 50 |
| Zinc gluconate | 160 |
| Extrait vin (20 % OPC) | 300 |
| Glycérine | 150 |
| stéarate de magnésium | 0,02 |
| Eau | QSP 900 mg |

### Exemple 4

### CAPSULE

| | **mg/capsule** |
|---|---|
| Taurine | 50 |
| Zinc gluconate | 160 |
| Extrait vin (20 % OPC) | 300 |
| Glycérine | 150 |
| stéarate de magnésium | 0,02 |
| Complexe vitaminique | QSP *?* |
| Eau | QSP 900 mg |

| | |
|---|---|
| * Le complexe vitaminique comporte, 60 mg de vitamine C, 50 µg de sélénium, 15 mg de vitamine E, 10 mg de zinc et 3 mg de lycopène. | |

### Exemple 5

### FORMULATION DE TYPE DRAGEE

| | **mg/dragée** |
|---|---|
| Taurine | 50 |
| Extraits pépins de raisin (40 % OPC) | 100 |
| Extraits Thé vert (30 % Catechines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

### Exemple 6

### FORMULATION DE TYPE DRAGEE

| | **mg/dragée** |
|---|---|
| Extraits pépins de raisin (40 % OPC) | 100 |
| Extraits thé vert (30 % Catéchines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 2 fois par jour.

### Exemple 7

### FORMULATION DE TYPE DRAGEE

| | **mg/dragée** |
|---|---|
| Taurine | 50 |
| Extraits pépins de raisin (40 % OPC) | 50 |
| Extraits thé vert (30 % Catéchines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

### Exemple 8

### GELULE GELATINE VEGETALE OU ANIMALE

| **Principe actif** | **mg/gélule** |
|---|---|
| Extrait pépins de raisin (40 % OPC) | 50 |
| Extrait thé vert (30 % Catéchines) | 175 |
| Amidon | 128 |
| Stéarate de magnésium | 2,5 |

On peut prendre une à quatre gélules par jour.

### Exemple 9

### GELULE GELATINE VEGETALE OU ANIMALE

| **Principe actif** | **mg/gélule** |
|---|---|
| Taurine | 80 |
| Extrait pépins de raisin (40 % OPC) | 50 |
| Extrait thé vert (30 % Catéchines) | 175 |
| Amidon | 128 |
| Stéarate de magnésium | 2,5 |

On peut prendre une à quatre gélules par jour.

### Exemple 10

### GEL UNIDOSE

| **Principe actif** | **% pds** |
|---|---|
| Taurine | 4 |
| Extraits pépins de raisin (40 % OPC) | 4 |
| Extrait thé vert (30 % Catéchines) | 6 |
| Levure enrichie en zinc (22,75 % Zn) | 2 |
| | |

| **Excipient** | |
|---|---|
| Rhodigel™ | 2,3 |
| Extrait Cacao | 20 |
| Sorbate de potassium | 0,05 |
| Benzoate de sodium | 0,05 |
| Eau | QSP 100 |

On prend 200 à 400 ml de gel par jour.

### Exemple 11

### GEL UNIDOSE

| **Principe actif** | **% pds** |
|---|---|
| Extrait pépins de raisin (40 % OPC) | 4 |
| Extrait thé vert (30 % Catéchines) | 10 |
| Huile de pépin de cassis | 10 |
| | |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On prend 200 à 400 ml de gel par jour.

### Exemple 12

### CAPSULE

| | **mg/capsule** |
|---|---|
| Taurine | 50 |
| Zinc gluconate | 60 |
| Extrait vin (20 % OPC) | 300 |
| Glycérine | 150 |
| stéarate de magnésium | 0,02 |
| Eau | QSP 900 mg |

On prend une à quatre capsules par jour.

### Exemple 13

### POUDRES

Un extrait de vin 1,8 g apportant jusqu'à 540 mg de polyphénols totaux ((dont 360 mg d'OPC)), jusqu'à 120 mg de taurine, 0,01 g édulcorant Goldblend, 0,4 g Arôme FRAM0584 et maltodrextrine 4 g a été utilisé sous forme de poudre à diluer dans l'eau, dans un produit laitier ou incorporé dans une barre alimentaire céréales / fruits à consommer chaque jour.

On a utilisé dans les mêmes conditions :
- Un mélange d'extraits de Vitis Vinefera et/ou d'un de ses produits de biotechnologie (jus raisin, vin...) apportant la même quantité de polyphénols totaux en association avec une source de protéines naturelles riches en taurine apportant une quantité de taurine de 150mg/j.
- Un extrait de Camellia Simensis ou de Théobroma Cacao apportant la même quantité de polyphénols totaux, en association avec une source de protéines naturelles riche en taurine apportant la même quantité de taurine.

### Exemple 14

On adjoint à la formulation gel de l'exemple 10 un complexe vitaminique comportant 120 mg de vitamine C, 100 µg de vitamine E, 20 mg de zinc et 6 mg de β-carotène, dans 200 ml de gel de l'exemple 2 et 60 mg de vitamine C, 50 µg de vitamine E, 10 mg de zinc et 3 mg de β-carotène dans une dragée de l'exemple 7.

### Exemple 15

Dans les formulations de l'exemple 14, on remplace le β-carotène par le lycopène.

### Exemple 16

### GEL UNIDOSE

| **Principe actif** | **% pds** |
|---|---|
| Taurine | 4 |
| Extraits pépins de raisins à 40 % OPC | 4 |
| Extrait de thé vert à 30 % Catéchines | 6 |
| Huile de pépins de cassis | 10 |
| | |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On peut prendre une dose de 200 à 400 ml par jour.

### Exemple 17

### CAPSULE

| | **mg/capsule** |
|---|---|
| Taurine | 50 |
| Zinc gluconate | 60 |
| Extrait vin (20 % OPC) | 200 |
| Huile de pépins de cassis | 300 |
| Glycérine | 150 |
| stéarate de magnésium | 0,02 |
| arôme naturel | |
| Eau | QSP 900 mg |

On peut prendre une à trois de ces capsules par jour.

### Exemple 18

### FORMULATION DE TYPE DRAGEE

| | **mg/dragée** |
|---|---|
| Taurine | 50 |
| Extraits pépins de raisin (40 % OPC) | 50 |
| Extraits thé vert (30 % Catéchines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

### Exemple 19

On adjoint à la formulation de l'exemple 16, un complexe vitaminique comportant 120 mg de vitamine C, 100 µg de vitamine E, 20 mg de zinc et 6 mg de β-carotène, pour 200 ml de gel.

### Exemple 20

On adjoint à la formulation de l'exemple 16, un complexe vitaminique comportant 120 mg de vitamine C, 100 µg de vitamine E, 20 mg de zinc et 6 mg de lycopène pour 200 ml de gel.

### Exemple 21

On adjoint à la formulation de l'exemple 19, un complexe vitaminique comportant 60 mg de vitamine C, 50 µg de vitamine E, 10 mg de zinc et 3 mg de lycopène pour une dragée.

### Exemple 22

### FORMULATION DE TYPE COMPRIME

| **Principe actif** | **Mg/Comprimé** |
|---|---|
| Taurine | 75 |
| Extraits pépins de raisin (40 % OPC) | 75 |
| Extraits thé vert (30 % Catéchines) | 187,5 |
| Zinc gluconate ( 14,3% de zinc) | 52,3 |
| | |
| **Excipient** | **Qsp pour 1 g** |

Ce type de comprimé est pris 2 fois par jour.

### Exemple 23

Deux groupes de 36 femmes âgées de 18 à 40 ans environ ayant les cheveux fins, mous et séborrhéiques ont absorbé pendant 6 mois :
- soit la formule capillaire de composition comme suit :

| | mg |
|---|---|
| Taurine | 150 |
| Extrait de thé vert à (30 % Catéchines) | 375 |
| Extrait de pépins de raisins (40 % OPC et 20 % catéchines) | 150 |
| Sulfate de zinc (22,75 %) | 15* |

| | |
|---|---|
| **exprimé en poids de zinc* | |

- soit un placébo, comprimé d'aspect identique à base de maltodextrine.

L'effet du traitement est examiné par auto-évaluation, et en appliquant trois coups de peigne à T0, T3 mois et T6 mois.

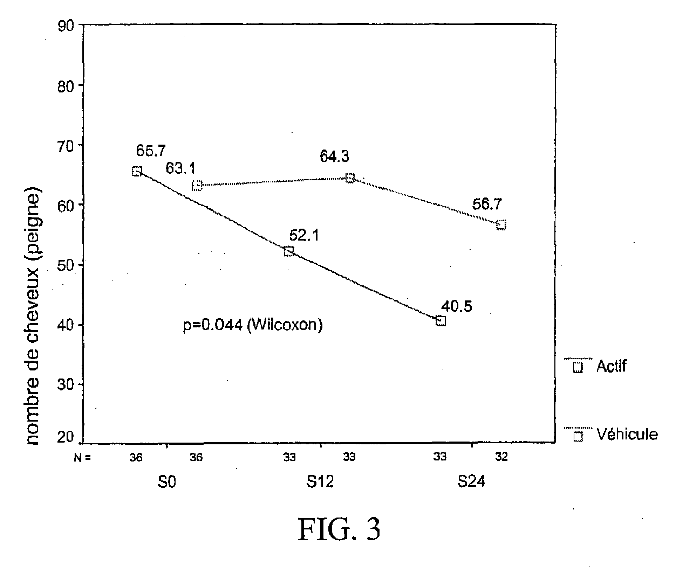

On note une diminution régulière du nombre de cheveux sur le peigne dans le groupe traité, cette différence étant statistiquement significative, comparativement au groupe à 6 mois ayant reçu le placebo. Les résultats sont présentés en figure 3.

## Revendications

1. Utilisation de polyphénol(s) choisi(s) parmi les flavonols, anthocyanines, flavanols, proanthocyanidines et flavanones, et stilbènes pour la préparation d'une composition orale utile dans le traitement ou la prévention des désordres de l'unité pilo-sébacée.

2. Utilisation selon la revendication 1 desdits polyphénol(s) et d'acide(s) gras choisi(s) parmi les acides gras polyinsaturés essentiels n-6 et n-3, comportant entre 18 et 22 atomes de carbone, ainsi que leurs esters, et leurs mélanges et/ou d'un extrait en comportant, pour la préparation de ladite composition.

3. Utilisation selon la revendication 1 ou 2, pour la préparation d'une composition orale utile pour réduire ou prévenir la chute des cheveux.

4. Utilisation selon les revendications 1, 2 ou 3, pour la préparation d'une composition orale utile pour réduire ou prévenir l'impact de la testostérone sur l'unité pilo-sébacée.

5. Utilisation selon l'une des revendications 1 à 4, **caractérisée en ce que** les polyphénols sont choisis parmi la catéchine, l'épicatéchine, l'épigallocatéchine-3-O-gallate, l'épigallocatéchine, l'épicatéchine-3-gallate, les procyanidines proanthocyanidines et leurs mélanges.

6. Utilisation selon l'une des revendications 1 à 5, **caractérisée en ce que** la dose journalière de polyphénol(s), est comprise entre 0,5 et 2000 mg/j.

7. Utilisation selon l'une des revendications 1 à 6, **caractérisée en ce que** la dose journalière de polyphénols est de l'ordre de 0,5 à 1000 mg/j.

8. Utilisation selon l'une des revendications 2 à 7, **caractérisée en ce que** le ou les acides gras sont choisis parmi l'acide linoléique, l'acide γ-linolénique, l'acide linolénique, l'acide stéaridonique, la crocétine et l'acide 5, 8, 11, 14 éicosatétrainoïque et leurs mélanges.

9. Utilisation selon l'une des revendications 2 à 8, **caractérisée en ce que** la dose journalière d'acides gras, est comprise entre 0,5 et 3500 mg/j, de préférence entre 5 et 1500 mg/j.

10. Utilisation selon l'une des revendications 2 à 9, **caractérisée en ce que** la dose d'acides gras n-6 est comprise entre 0,5 et 2600 mg/j, de préférence 5 à 1200 mg/j.

11. Utilisation selon l'une des revendications 2 à 10, **caractérisée en ce que** la dose d'acides gras n-3 est comprise entre 0,5 et 2500 mg/j, de préférence 5 à 360 mg/j.

12. Utilisation selon l'une des revendications 1 à 11, **caractérisée en ce que** la composition orale comporte en outre l'un au moins des compléments choisis parmi les vitamines C et E, du zinc ou ses sels, le sélénium et au moins un caroténoïde, de préférence un caroténoïde choisi parmi le β-carotène, le lycopène, la zéaxanthine et la lutéine.

13. Procédé cosmétique de traitement et de prévention des désordres de l'unité pilo-sébacée par l'administration orale d'au moins un acide gras, un polyphénol ou d'un extrait en comportant éventuellement en association avec de la taurine et/ou de l'hypotaurine et/ou leurs sels acceptables.

14. Procédé selon la revendication 13 dans lequel lesdits polyphénols sont choisis parmi les flavones, flavonols, isoflavones, anthocyanines, flavanols, proanthocyanidines et flavanones, et stilbènes et lesdits acides gras les acides gras sont choisis parmi l'acide linoléique, l'acide γ-linolénique, l'acide linolénique, l'acide stéaridonique, la crocétine et l'acide 5, 8, 11, 14 éicosatétrainoïque et leurs mélanges.

15. Procédé selon la revendication 13 ou 14, dans lequel on administre, au moins un acide gras à une dose de 0,5 à 5400 mg/j et au moins un polyphénol à une dose de 0,5 à 2000 mg/j et éventuellement la taurine, l'hypotaurine ou leurs sels acceptables à une dose de 0,5 à 4000 mg par jour en équivalent taurine.
